# EUROPEAN PATENT APPLICATION

(11) **EP 2 431 001 A1**
(43) Date of publication of application: **21.03.2012**
(21) Application number: 10290495.0
(22) Date of filing: 16.09.2010
(51) Int. Cl.: A61B 18/20, A61B 18/00, A61B 18/22

(54) **Laser-based lipolysis**

(71) Applicant: Dornier MedTech Laser GmbH, 82234 Wessling (DE)
(72) Inventor: Rheinwald, Markus, Dr., 86916 Kaufering (DE); Milleret, René, Dr., 34000 Montpellier (FR)
(74) Representative: Grünecker, Kinkeldey, Stockmair & Schwanhäusser

(57) **Abstract**

The invention provides a system and method for percutaneous delivery of a constant thermal energy to a treatment site resulting in a reduction of adipose tissue with little or no damage to other adjacent tissue with no side effects, good skin contraction or tightening and better patient recovery.

## Description

### I. Background

The weight and the body contour of human individuals are largely influenced by the amount and volume of adipose tissue in subcutaneous layers. For selected individuals, a higher volume of adipose tissue in them represents a greater deviation from a desirable body appearance. Therefore, recent years have seen an increased desire by those individuals to improve their weight or their body contour or both by getting rid of substantial portions of their body fat, which is contained in solid or gel-like form within adipose cells.

An early proposed method to address this desire was a procedure called liposuction in which the bodily fat was removed from the body by sucking it out through thin tubes or needles using underpressure. In a subsequent step of development, vibration of the needle (or tube) tip was used to mechanically break the membranes of the adipose cells, to enable removal thereof. This approach has been problematic because the layer of fat tissue underneath the skin is not all homogeneous within itself.

Collageneous septae connect the tissue layers on top and underneath the layer of fat tissue and, therefore, cross the layer of fat tissue. These septae cause increased resistance to the movement of the tube or needle used for sucking out the fat whenever the tip of the tube or needle is pushed against such a septum. In order to maintain a certain constancy in the speed of movement of the tube or needle, the operator usually applies an increased force for the movement, which is sufficient for him to be able to overcome the increased resistance without significant change of the movement. This creates some degree of violence in the movements necessary for the completion of the procedure, which causes procedural trauma to the treatment site. This trauma is associated with considerable blood loss peri-operatively and generation of pain to the patients post-operatively, which results in prolonged recovery times. In addition, the skin covering the volumes of fatty tissue being removed remains stretched post-operatively, in spite of volumes of fat being removed. This results in an unfavorable look of skin laxity over the body zones treated.

However, these early liposuction techniques were advantageous because they defined important procedural steps that have generally been maintained in the following stages of technical and methodological development:
1. The application of tumescent anesthesia for a. effecting topical reduction of pain sensation during the procedure, b. spatially buffering adjacent tissue structures from mechanical damage, and c. creating a medium for the sputtered debris of adipose cells and the contained fat to be sucked out with underpressure through the needle or tube.
2. The use of long needles or tubes for a. needing only a small hole for access to the tissue layer of interest, hence being minimally invasive, and b. being able at the same time to reach out to tissue regions far out in lateral distance from the entrance hole yet in the same target layer.
3. Using those needles or tubes to scan over an area of interest within the target layer in the adipose tissue. The primary scan movement is a forth-and-back movement of the needle or tube along its longitudinal extension to sequentially access a target amount of individual target volumes within the target tissue layer along the line of the longitudinal movement, the line having an extension from the minimal distance from the entry point of the needle or tube through the skin into the target layer to the maximum distance to this point. The minimal distance is determined by the minimal length of the portion of the tube or needle that is required to stay inside the target tissue layer such as not to inadvertently slip out of the entry point. The maximum distance is determined by a. the length of the tube or needle used and b. the extension of the target scan pattern in that direction with respect to the spatial arrangements of scan patterns in order to cover the target area to be treated to the degree that is maximally achievable. During that movement and with the underpressure applied to the tip of the tube or needle, the volume of fat in direct contact with the tip of the tube or needle at a given time will be sucked into the tube or needle, hence to outside the body.
   The secondary scan movement is to displace the line along which the tube is moved forth and back within the primary scan movement with an incremental azimuthal angle around the entry point within the target layer after one cycle of the primary scan movement (one movement forth and one movement back of the tip of the needle or tube). The degree of the incremental azimuthal angle applied in an individual patient is determined by the operator depending on the amount of fat to be removed, the rate at which the fat can be removed through the tube or needle, and the maximum distance of the line of the primary scan movement to the entry point that would then define a maximum radius. Complete coverage of the target area by the scan pattern is then achieved if the diameter of interaction of the tip of the tube or needle with the bodily fat equals the maximum radius multiplied with the incremental radian azimuthal angle of the secondary scan movement. A series of secondary scan movements would finally result in a fan-shape for the complete scan pattern. Overall azimuthal angles for the fan patterns are usually in the 30 to 60 degrees range.
4. A wrapping of the treated body zone post-treatment in order to support the healing process.

It was then proposed to generate heat at the tip of the elongate applicator by applying different forms of energy. Forms of energy proposed were mostly electromagnetic radiation in the visible and infrared ranges of the wavelength spectrum. The application of energy at the distal end of the elongate applicator would lead to the absorption of the energy in a tissue volume adjacent to that distal end; the energy absorbed would in turn be transformed into heat energy contained in that tissue volume. The amount of heat energy deposited would result in an increase of the temperature of that tissue volume depending on the size of the volume and the specific heat capacity of the tissue in that volume. The heat energy applied would in turn result in the following:
1. Destruction of the membrane of the adipocytes. This would set free the fat from within the adipocytes to the intercellular space from which it can be evacuated more easily, either by suction to outside the body or by bodily self-cleaning processes.
2. Liquefaction or "melting" of the fat, hence making it more viscous which would ease the process of evacuation. The aqueous fluid of the tumescent anesthetic is enveloping the target layer of tissue treated and will over a specific time act as a heat sink to the local heating of the target tissue volume, which target volume will cool down again by dissipation of the heat. However, once liquefied and at the same time being surrounded with the aqueous tumescence fluid, the fat would emulsify with that liquid and hence remain easy to be evacuated after having cooled down.
   These first two steps have also led to a variation of heat-assisted reduction of body fat, as indicated above already (in 1.): Instead of evacuating the dissolved fat through the tube or needle during the procedure, it was possible to leave the dissolved fat inside the body and wait for bodily self-cleaning processes, potentially supported by various forms of mechanical manipulation of the body zone from outside (e.g., massage, lymphatic drainage). This natural process of fat removal would lead to a more homogeneous appearance of the treated body zone, however, it would take additional procedures for the patient to undergo (e.g., the massage or lymphatic drainage) and to wait for an additional time for the final result. The method of fat evacuation to be applied would depend on practicability issues and the preferences of the operator and/or the patient.
3. Application of heat energy to the layer of skin being situated on top of the target layer of fat tissue. This would tighten that layer of skin that was stretched by the underlying volume of fatty tissue pre-operatively. This would result in a reduced skin laxity post-operatively, and ideally in a restitution of the skin tightly covering the reduced volume in the body zone treated.
4. With its highest density concentrated around the very tip of the tube or needle, the heat energy would also help the fiber tip to glide more smoothly around the septae between the tissue layers on top and underneath the target layer of fat tissue. This would reduce the resistance caused by these septae against the movement of the tip of that element. Consequently, the operator can reduce the force he is applying for the realization of the longitudinal movement, and hence can take out some of the "operational violence," reducing the procedural trauma to the patient. Furthermore, the heat applied to the septae would lead to their slight contraction going in line with the reduction of height of the fat layer. In addition, the heat applied would have a hemostatic effect to the operational traumata, reducing the overall blood loss. The reduced procedural mechanical trauma and reduced bleeding together would represent a milder procedure for the patient and a faster recovery time.

However, these new methods were not free of flaws. The way the heat energy is applied to the tissue causes some inhomogeneities in the individual energies deposited to a specific volume of tissue, hence leading to inhomogeneous results, which are either: a. overtreatment by applying too much energy, resulting in an increased rate of side-effects, some of them potentially irreversible, or b. under treatment of specific volumes in the attempt to avoid overtreatment of others, resulting in an unsatisfactory result as to the desired effect of the treatment. The inhomogeneities can be categorized into intrinsic and extrinsic ones. The inhomogeneities include the following, with items 1-2 being intrinsic inhomogeneities and items 3-5 being extrinsic inhomogeneities:
1. As the secondary scanning movement is represented by an incremental angulation of the primary longitudinal movement of the tube or needle, more overlap of target tissue volumes will naturally occur for the volumes lying more proximal to the entry point of the tube or needle.
2. A complete target zone on the body to be treated has in most cases a more complex form than just a fan. So, that complete target zone must be covered by laterally putting together multiple fan-shaped scan patterns to cover an area as similar in shape and extension as possible to it. However, a set of multiple fan-like patterns is rather prone to see overlaps as only two sides of the pattern are clearly defined by the first and the last line (primary scan movement) of that very pattern.
3. Inhomogeneities in the angular stepping (secondary scan movement).
4. Inhomogeneous lengths between individual lines of forth-and-back movements (primary scan movement).
5. Inhomogeneities in the speed of the forth-and-back movement (primary scan movement) caused by a. the doctor himself, as it is a non-standardized manual movement, b. the natural acceleration and deceleration around the turning points and c. by the inhomogeneous resistance the tip of the needle or tube is experiencing due to the inhomogeneous nature of the tissue (adipose vs. collagenous; native vs. processed).

To overcome these inhomogeneities, various methods of treatment feedback have been introduced to indicate various parameters to the operator and/or the energy supplying device:
1. Indication to the operator of the temperature of the tissue to be treated, which is either on natural tissue temperature, if not treated yet, or on an elevated temperature, if pretreated within the same session, but the heat energy not dissipated completely yet. This would make the respective tissue more sensitive to scan pattern overlaps resulting in overtreatment by overheating.
2. Indication to the operator on whether the needle has covered a specific point within the layer to be treated such that the operator would spatially avoid overlapping.
3. Indication to the laser device of the temperature in the surrounding of the tip of the tube or the needle in order to adjust the power of the energy source as to only supply the amount of power needed to reach a certain target temperature.

However, those methods cope with the inhomogeneities of the procedure in a limited way only. The description of exemplary embodiments of the invention will show in more detail what surprisingly different findings were made when applying an apparatus according to the exemplary embodiments and how this is an improvement over the traditional methods described above.

### II. Detailed Description of Exemplary Embodiments

In certain exemplary embodiments, a laser energy emitting device is coupled to a fiber having a working end. The device regulates the emitted laser energy such that the thermal energy emitted at the working end is maintained at a constant temperature that is sufficiently high enough to emulsify or liquefy the adipose tissues and to deform the septae, yet low enough not to irreversibly damage or harm the adjacent tissue structures, including the septae.

For example, radiation may be emitted by the pyrolytic glowing of biological tissue at the tip of the working end of the fiber. The radiation may be received by the distal end of the fiber, guided back to a radiation detector situated in the laser energy emitting device, and detected in a spectral region of between 0.3 and 0.9 µm so as to regulate the laser energy emitted such that the thermal energy emitted at the working end is maintained at a constant temperature. An exemplary device that may be useful for this application is described in U.S. Patent No. 5,098,427 ("the '427 patent").

Having a constant temperature that is sufficiently high enough to emulsify or denature the adipose tissues (as opposed to rupturing the membranes of the cells forming the adipose tissues) would achieve the objective of reducing the unwanted fatty tissue in humans or the desired contouring procedure. However, the constant temperature should also be high enough to only deform the septae, not to irreversibly damage or harm the septae. This will allow the epidermis or the skin at the treatment area to properly contract after the procedure without any side effects, such as negative effects on pigmentation, etc. Additionally, the constant temperature maintained at the working end of the fiber should also be not so high as to irreversibly damage or harm the adjacent tissue structures, such as the dermis or epidermis.

In certain exemplary embodiments, the device is designed to allow different constant temperatures for different applications. For example, temperatures may vary depending on the type of bodily tissue being treated.

In certain exemplary embodiments, a person may be treated using the device by:
(a) injecting tumescent anesthesia in the intended treatment area, (b) applying thermal energy using the system described above, and (c) wrapping the treatment site (e.g., with bandages).

For example, the process of energy deposition and heat generation may include the following steps:
1. Generation of electromagnetic energy (e.g., light) at a certain power within an appropriate console, such as a laser.
2. Guiding the electromagnetic energy from the console to the target tissue via an appropriate guiding means, such as an optical light guide.
3. Emission of the electromagnetic energy from the distal end of the guiding means and targeting it to the tissue volume to be treated. In the case of laser liposuction or laser lipolysis, the distal end of the optical light guide generally will be in contact with the target tissue volume inside the body.
4. Absorption of electromagnetic energy by specific tissue components (such as water, hemoglobin, collagen, fat, etc.) within the volume irradiated. The size of the volume within which the electromagnetic energy will be absorbed depends on the absorption properties of that volume (e.g., character, properties, and concentration of absorbants contained in the volume).
5. Transformation of the electromagnetic energy absorbed into heat energy carried by the bodily absorbants.
6. Transportation of the heat energy generated out of the transformation from the electromagnetic energy to volumes adjacent to the absorbants; the process being ruled by various heat energy transport mechanisms; the volumes including tissue in most cases, but also e.g., the tumescent medium.
7. Changes to the biological tissue on both microscopic and macroscopic levels due to the heat energy applied there, the amount, speed and character of change being ruled by a function of the amount of heat energy the tissue is exposed to and the time it is exposed to the heat.
In certain exemplary embodiments, steps 6 and 7 can be parallel processes.

Shortly after the start of the treatment procedure, the following will happen: Due to the high density of heat energy generated around the distal tip of the laser fiber, the tissue surrounding it will achieve such high temperatures that it will carbonize and stick to the fiber tip. After its generation, this charred tip will absorb almost all the laser light energy coming to it from the laser side, and the light energy will be transformed into heat energy within that tip. In certain exemplary embodiments, the tip has a small volume of about 1 mm³ or less and will therefore achieve very high temperatures able to emit light with a continuous spectrum in the visible light spectrum. With the fiber tip moved along longitudinal lines, the heat energy generated in the tip will be transported into the volumes surrounding this line, continuously taking up the heat energy which is generated by irradiating the charred fiber tip from the laser side. Then, this heat energy arriving at those volumes will cause the change of the tissue characteristics. So, the processes mentioned above would change from step 3:
3. Irradiation of the charred tissue stuck to the distal tip of the fiber with the light energy coming out of the laser.
4. Absorption of the light energy within that volume of charred tissue sticking to the distal tip.
5. Transformation of the light energy into heat energy.
6. Transportation of that heat energy to volumes adjacent to the volume of charred tissue at that given time (with simultaneous longitudinal movement of that volume of charred tissue).
7. Changes to the biological tissue on both microscopic and macroscopic levels due to the heat energy applied there, the amount, speed and character of change being ruled by a function of the amount of heat energy the tissue is exposed to and the time it is exposed to the heat.

Applying a laser energy emitting device incorporating the fibertom mode as described in the '427 patent may sense the thermal feedback in an early stage of those steps, i.e., within steps 3 to 5. Those steps may occur within a very short time frame, i.e., up to about 20 ms, more preferably up to 10 ms. The other temperature feedback mechanisms are only seeing the thermal effects at steps 6 or 7, which is longer than this time, and potentially too long to have a beneficial effect for the immediate incremental power needed to be supplied to achieve a constant temperature at the fiber tip. A fast feedback will be beneficial in avoiding excessive temperatures at the fiber tip which could be harmful to tissue volumes including fat, skin, and septae, potentially resulting in irreversible damage.

Methods 1 and 2 would address the more static inhomogeneities of listed previously as items 2 and 4. Method 3 would largely depend on the time cycle needed for the tissue to reach the end temperature responsible for the final effect plus the reaction time for the thermal sensor plus the reaction time for the energy source to adjust the power to the appropriate value. Temperature sensors on the tip of the needle or the tube would have a longer reaction time, such that inhomogeneities according to items 1-4 listed previously are not flattened fast enough due to the speed of the movement required to timely complete the procedure. In addition, they cannot address inhomogeneities according to item 5 listed previously, which would require adapting the power almost instantaneously in order to resect collagenous structures different from the adipose tissue within the target layer.

An additional device is used to add accuracy and objectivity to the operators' practice of feeling the temperature of the tissue treated at the outside of the body through the skin and to potentially complement the fibertom mode as a means for thermal feedback on a short time scale. Such a device can take the form of an accessory provided on the operator's finger with a temperature indicator, such as a thermometer or a sheath located on the skin covering the treatment area with a visual indicator for the operator. This will provide thermal feedback on the temperature of the tissue volume being treated rather than the fiber tip as covered by the fibertom feature.

## Claims

1. A laser-based lipolysis system for reducing adipose tissue of a patient while not or only minimally harming adjacent tissue structure, in particular septae, the system comprising:
a laser light emitting console,
an optical light guide coupled to said console and having a working end, and
a regulation means for regulating the emitted laser energy to be high enough for reducing the adipose tissue, and to be low enough for not or only minimally harming adjacent tissue structures,
wherein the regulation means is situated in the laser light emitting console.

2. A laser-based lipolysis system comprising:
a laser light emitting console,
an optical light guide coupled to said console and having a working end, and
a regulation means for regulating the emitted laser energy to be high enough for reducing the adipose tissue, and to be low enough for not or only minimally harming adjacent tissue structures,
wherein the regulation means is situated in the laser light emitting console, and wherein the system is used for reducing adipose tissue of a patient while not or only minimally harming adjacent tissue structure, in particular septae.

3. The laser-based lipolysis system of claims 1 or 2, wherein the regulation means comprises a radiation detector for detecting back guided radiation in a spectral region of between 0.3 and 0.9 µm.

4. The laser-based lipolysis system of one of the preceding claims, wherein the regulation means comprises a radiation detector for detecting radiation of a pyrolytic glowing of biological tissue on the working end of the optical light guide.

5. The laser-based lipolysis system of one of the preceding claims, wherein the regulation means is a regulation means for keeping a temperature of thermal energy emitted at the working end of the optical light guide constant.

6. The laser-based lipolysis system of one of the preceding claims, wherein the regulation means has a short time frame feedback mechanism, preferably for short time frames of up to 20 ms, more preferably for short time frames of up to 10 ms.

7. The laser-based lipolysis system of one of the preceding claims, for deforming the septae.

8. The laser-based lipolysis system of one of the preceding claims, for reducing adipose tissue while not or only minimally harming adjacent tissue structures of dermis or epidermis.

9. The laser-based lipolysis system of one of the preceding claims, for denaturing or emulsifying adipose tissue by laser light.

10. The laser-based lipolysis system according to one of the preceding claims comprising a tissue temperature sensing device for sensing a temperature of treated tissue on the outside of a patient's body.

11. The laser-based lipolysis system of claim 10, wherein the temperature sensing device complements the regulation means.

12. The laser-based lipolysis system of claims 10 or 11, wherein the temperature sensing device takes the form of an accessory that can be provided on an operator's finger.

13. The laser-based lipolysis system of claims 10 to 12, wherein the temperature sensing device comprises a temperature indicator, preferably a thermometer or a sheath that can be located on skin covering the treatment area.
